# FASCICULE DE BREVET EUROPEEN

(11) **EP 1 299 102 B1**
(45) Date de publication et mention de la délivrance du brevet: **19.04.2006**
(21) Numéro de dépôt: 01940687.5
(22) Date de dépôt: 05.06.2001
(51) Int. Cl.: A61K 31/425, A61P 3/00

(54) **UTILISATION DU RILUZOLE OU SES SELS POUR LA PREVENTION ET LE TRAITEMENT DE L'ADRENOLEUCODYSTROPHIE**
VERWENDUNG DES RILUZOLS ODER DESSEN SALZEN ZUR PHOPHYLAXE UND BEHANDLUNG DER ADRENOLEUKODYSTROPHIE
USE OF RILUZOLE OR ITS SALTS FOR PREVENTING AND TREATING ADRENOLEUKODYSTROPHY

(30) Priorité: 05.06.2000 FR 0007162
(43) Date de publication de la demande: 09.04.2003
(73) Titulaire: Aventis Pharma S.A., 92160 Antony (FR); INSTITUT NATIONAL DE LA SANTE ET DE LA RECHERCHE MEDICALE (INSERM), 75654 Paris Cédex 13 (FR)
(72) Inventeur: AUBOURG, Patrick, F-92100 BOULOGNE-BILLANCOURT (FR); DIB, Michel, F-75013 PARIS (FR); STUTZMANN, Jean-Marie, F-94440 VILLECRESNES (FR)
(74) Mandataire: Rousseau, Pierrick Edouard
(86) Numéro de dépôt international: PCT/FR2001/001729
(87) Numéro de publication internationale: WO 2001/093802

(56) Documents cités:
- WO-A-99/51097
- VOGELS O.J.M. ET AL: "Decreased striatal dopamine-receptor binding in sporadic ALS: Glutamate hyperactivity?." NEUROLOGY, (1999) 52/6 (1275-1277)., XP000982459
- DATABASE BIOSIS [en ligne] BIOSCIENCES INFORMATION SERVICE, PHILADELPHIA, PA, US; 1999 SINGH I ET AL: "Lovastatin therapy for X-linked adrenoleukodystrophy (X-ALD)." Database accession no. PREV199900150372 XP002161011 & JOURNAL OF NEUROCHEMISTRY, vol. 72, no. SUPPL., 1999, page S19 30th Annual Meeting of the American Society for Neurochemistry;New Orleans, Louisiana, USA; March 14-17, 1999 ISSN: 0022-3042

## Description

La présente invention concerne l'utilisation du riluzole ou un de ses sels pharmaceutiquement acceptables pour la prévention et le traitement de l'adrénoleucodystrophie.

Le riluzole (2-amino-6-trifluorométhoxybenzothiazole) est commercialisé pour le traitement de la sclérose latérale amyotrophique (Rilutek^{R}). Ce composé est également utile comme anticonvulsivant, anxiolytique et hypnotique (EP50551), dans le traitement de la schizophrénie (EP305276), dans le traitement des troubles du sommeil et de la dépression (EP305277), dans le traitement des désordres cérébrovasculaires et comme anesthésique (EP282971), dans le traitement des traumatismes spinaux, crâniens ou crânio-spinaux (WO94/13288), comme radiorestaurateur (WO94/15600), dans le traitement de la maladie de Parkinson (WO94/15601), dans le traitement du neuro-sida (WO94/20103), dans le traitement des maladies mitochondriales (WO95/19170).

L'adrénoleucodystrophie (ADL) liée à l'X est la plus fréquente des maladies génétiques de la myéline qui se caractérise par une démyélinisation progressive du système nerveux central, une insuffisance surrénale et une accumulation modérée (X 3 à 5) des acides gras à très longue chaîne (AGTLC) dans la plupart des tissus, y compris le cerveau et la moelle épinière. Cette accumulation d'AGTLC résulte d'un déficit de leur β-oxydation dans le péroxysome.

Les mécanismes conduisant dans l'ALD à une démyélinisation et une perte des oligodendrocytes restent peu compris. Une des possibilités est que l'accumulation des AGTLC conduise à une déstabilisation des membranes myéliniques ou à un dysfonctionnement des récepteurs situés à la surface des oligodendrocytes les rendant plus sensibles aux signaux de mort cellulaire programmée (apoptose). La mort des oligodendrocytes pourrait aussi résulter de la production de cytokines (en particulier TNF-α) par les macrophages activés qui sont présents dans les lésions inflammatoires cérébrales d'ALD.

Dans une étude de la fragmentation de l'ADN, par la méthode TUNEL et l'expression de la caspase-3, 50% des oligodendrocytes de cerveaux de patients ALD présentent des signes de mort cellulaire par apoptose et l'intensité des phénomènes apoptotiques est corrélée à l'intensité de la démyélinisation.

Une mort des oligodendrocytes par apoptose a été récemment démontrée dans 2 autres modèles murins de maladie génétique de la myéline : la souris twitcher (modèle de la maladie de Krabbe) et différents modèles déficients en protéine protéolipide, PLP (souris jimpy, souris msd, rat md).

Il a maintenant été trouvé que le riluzole éventuellement sous forme de sel pharmaceutiquement acceptable réduit la mort des oligodendrocytes par apoptose induite par le kainate et, de ce fait peut être utilisé dans la prévention et le traitement de l'adrénoleucodystrophie.

Le protocole utilisé est le suivant :

### Cultures enrichies en oligodendrocytes

Des oligodendrocytes sont obtenus à partir de cultures gliales primaires de moelles épinières selon la méthode décrite par Saneto et coll. (Neuchemistry, a practical approach, IRL Press, Oxford-Washington DC, p27-63 (1987)) légérement modifiée. Des moelles épinières de rats Wistar âgés de 1 jour sont disséquées dans des conditions stériles et séparées des méninges. Cinq à dix moelles épinières sont transferées dans du PBS (phosphate buffer saline : NaCl 137 mM, KCl 2,68 mM, Na₂HPO₄ 6,45 mM, KH₂PO₄ 1,47 mM) auquel on a ajouté 0,25% de trypsine. Le traitement enzymatique est arrêté par addition de milieu Dulbecco Eagle modifié (DMEM) auquel on a ajouté 10% de sérum foetal bovin (FBS). Une autre étape de dissociation est effectuée au moyen d'une pipette de 1 ml et la suspension cellulaire est filtrée. Les cellules sont collectées par centrifugation et étalées à une densité de 1,5-2x10⁶ cellules/25cm² de milieu de culture dans du milieu Dulbecco Eagle modifié (DMEM) auquel on a ajouté 10% de sérum foetal bovin. Après 3 jours in vitro, les cultures sont nourries chaque jour. Quand une monocouche visible est obtenue, les cultures sont agitées 2 heures à 37°C à 250 rpm, le milieu de culture est écarté et les flacons sont encore agités 22 heures. Le milieu contenant les cellules est incubé à 37°C dans des boites de pétri pendant 1 heure pour que les microglies s'attachent au plastique. Les oligodendrocytes sont collectés par centrifugation, étalés à une densité de 1,6x10⁶/cm² et maintenus dans du DMEM à 10% de FBS pendant 1 ou 2 jours. Le milieu est alors remplacé par du milieu L15 auquel on ajoute du bicarbonate de sodium (22mM), de la conalbumine (0,1 mg/ml), de la putrescine (0,1 mM), de l'insuline (5ug/ml) du sélénite de sodium (31 nM), du glucose (20 mM), de la progestérone (21 nM), de la pénicilline (100 IU/ml), de la streptomycine (100 µg/ml) et du sérum de cheval (2%). Les oligodendrocytes se différencient rapidement en 24 à 48 heures en phénotype mature et immature. Ces cultures montrent une pureté d'environ 95 % déterminée par l'immunoréactivité au galactocérébroside-C (Gal-C) et à la protéine gliale fibrilaire acide (GFPA).

A ces cultures, on ajoute ensuite du kainate en solution dans du PBS à différentes concentrations (0,01 mM à 1 mM), du riluzole en solution dans NaCl à 0,9 % et HCl à 0,001N, à différentes concentrations (0,01 mM à 10 mM) ou du solvant. Après 48 heures à compter du traitement, les oligodendrocytes vivants sont comptés.

### Comptage des cellules

Les cellules immunoréactives pour Gal-C et exhibant des procédés branchés plus longs que les diamètres de 2 cellules sont considérées globalement comme des oligodendrocytes immatures ou matures. Le nombre d'oligodendrocytes Gal-C (+) est réparti pour 2 comptages séparés des cellules marquées dans au moins 12 champs de 0,63 mm² au microscope 400x. Les valeurs sont exprimées comme le nombre de cellules par cm² ou comme le pourcentage du contrôle.

Les analyses statistiques sont effectuées en utilisant le test de Student (t-test).

Les résultats obtenus sont les suivants :

### Essai 1 : Effet du kainate sur les cultures d'oligodendrocytes

Exposition des cultures d'oligodendrocytes à des concentrations de 0,1 à 1mM de kainate pendant 48 heures.

| | Nombre d'oligodendrocytes % par rapport au contrôle ± écart type |
|---|---|
| Solvant seul | 100±17,4 |
| Kainate | |
| 0,01 mM | 108,2±25,2 |
| 0,1 mM | 76,3±27,9 |
| 0,5 mM | 25,3±9,3 |
| 1 mM | 22,9±7,1 |

Ces résultats démontrent que le kainate aux doses de 0,1 à 1 mM induit fortement la mort par apoptose des oligodendrocytes.

### Essai 2 : Recherche d'effet toxique du riluzole seul sur les cultures d'oligodendrocytes

| Exposition des cultures d'oligodendrocytes à des concentrations de 0,01mM à 10 mM de riluzole pendant 48 heures. | Nombre d'oligodendrocytes % par rapport au contrôle ± écart type |
|---|---|
| Solvant | 100±8,2 |
| Riluzole | |
| 0,01 mM | 99,6±15,7 |
| 0,1 mM | 94,3±17,3 |
| 1 mM | 107,1±9,5 |
| 10 mM | 86,9±16 |

Ces résultats démontrent que le riluzole aux doses de 0,01 mM à 10 mM n'a aucun effet toxique sur la survie des oligodendrocytes et qu'à la dose de 10 mM un léger effet toxique est observé.

### Essai 3 : Effet protecteur du riluzole sur la mort des oligocytes induite par le kainate

Exposition des oligodendrocytes au kainate à la dose de 1 mM et au riluzole aux doses de 0,01 mM à 10 mM.

| | Nombre d'oligodendrocytes % par rapport au contrôle ± écart type |
|---|---|
| Kainate 1000 | 22,9±7,1 |
| Kainate 1 mM + riluzole 0,01 mM | 48,7±7 |
| Kainate 1 mM + riluzole 0,1 mM | 65,2±9,2 |
| Kainate 1 mM + riluzole 1 mM | 69,4±12 |
| Kainate 1 mM + riluzole 10 mM | 56,3±6,9 |

Ces résultats démontrent que le riluzole diminue nettement la mort des oligodendrocytes par apoptose induite par le kainate et ceci même à la dose où un léger effet toxique du riluzole seul a été observé et peut ainsi être utilisé dans la prévention et le traitement de l'adrénoleucodystrophie.

Comme sels pharmaceutiquement acceptables du Riluzole peuvent être notamment cités les sels d'addition avec les acides minéraux tels que chlorhydrate, sulfate, nitrate, phosphate ou organiques tels que acétate, propionate, succinate, oxalate, benzoate, fumarate, maléate, méthanesulfonate, iséthionate, théophilline-acétate, salicylate, phénolphtalinate, méthylène-bis-β-oxynaphtoate ou des dérivés de substitution de ces dérivés.

Les médicaments sont constitués par au moins le Riluzole sous forme libre ou sous forme d'un sel d'addition avec un acide pharmaceutiquement acceptable, à l'état pur ou sous forme d'une composition dans laquelle il est associé à tout autre produit pharmaceutiquement compatible, pouvant être inerte ou physiologiquement actif. Les médicaments selon l'invention peuvent être employés par voie orale, parentérale ou rectale.

Comme compositions solides pour administration orale, peuvent être utilisées des comprimés, des pilules, des poudres (capsules de gélatine, cachets) ou des granulés. Dans ces compositions, le principe actif selon l'invention est mélangé à un ou plusieurs diluants inertes, tels que amidon, cellulose, saccharose, lactose ou silice, sous courant d'argon. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou d'autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont les suppositoires ou les capsules rectales qui contiennent, outre le produit actif, des excipients tels que le beurre de cacao, des glycérides semisynthétiques ou des polyéthylèneglycols.

Les doses dépendent de l'effet recherché, de la durée du traitement et de la voie d'administration utilisée ; elles sont généralement comprises entre 50 et 400 mg par jour par voie orale pour un adulte avec des doses unitaires allant de 25 à 200 mg de substance active.

D'une façon générale, le médecin déterminera la posologie appropriée en fonction de l'âge, du poids et de tous les autres facteurs propres au sujet à traiter.

Les exemples suivants illustrent des médicaments selon l'invention :

### Exemple A

On prépare, selon la technique habituelle, des comprimés dosés à 50 mg de produit actif ayant la composition suivante :
- Riluzole 50 mg
- Mannitol 64 mg
- Cellulose microcristalline 50 mg
- Polyvidone excipient 12 mg
- Carboxyméthylamidon sodique 16 mg
- Talc 4 mg
- Stéarate de magnésium 2 mg
- Silice colloïdale anhydre 2 mg
- Mélange de méthylhydroxypropylcellulose, polyéthylèneglycol 6000, dioxyde de titane (72-3,5-24,5) q.s.p. 1 comprimé pelliculé terminé à 245 mg

### Exemple B

On prépare, selon la technique habituelle, des gélules dosées à 50 mg de produit actif ayant la composition suivante :
- Riluzole 50 mg
- Cellulose 18 mg
- Lactose 55 mg
- Silice colloïdale 1 mg
- Carboxyméthylamidon sodique 10 mg
- Talc 10 mg
- Stéarate de magnésium 1 mg

### Exemple C

On prépare une solution injectable contenant 10 mg de produit actif ayant la composition suivante :
- Riluzole 10 mg
- Acide benzoïque 80 mg
- Alcool benzylique 0,06 cm3
- Benzoate de sodium 80 mg
- Ethanol à 95 % 0,4 cm3
- Hydroxyde de sodium 24 mg
- Propylène glycol 1,6 cm3
- Eau q.s.p. 4 cm3

L'invention concerne également l'utilisation du riluzole ou d'un de ses sels pharmaceutiquement acceptables pour la préparation d'un médicament utile pour la prévention ou le traitement de l'adrénoleucodystrophie.

L'invention concerne également le procédé de préparation de médicaments utiles dans la prévention ou le traitement de l'adrénoleucodystrophie consistant à mélanger le Riluzole ou les sels pharmaceutiquement acceptables de ce composé avec un ou plusieurs diluants et/ou adjuvants compatibles et pharmaceutiquement acceptables.

## Revendications

1. Utilisation du riluzole ou un de ses sels pharmaceutiquement acceptables pour la préparation d'un médicament pour la prévention et le traitement de l'adrénoleucodystrophie.

2. Utilisation selon la revendication 1 pour la préparation d'un médicament contenant de 25 à 200 mg de riluzole.

## Claims

1. Use of riluzole or one of its pharmaceutically acceptable salts for the preparation of a medicament for the prevention and the treatment of adrenoleukodystrophy.

2. Use according to Claim 1 for the preparation of a medicament containing 25 to 200 mg of riluzole.

## Patentansprüche

1. Verwendung von Riluzol oder einem seiner pharrmazeutisch annehmbaren Salze zur Herstellung eines Arzneimittels für die Prophylaxe und die Behandlung der Adrenoleukodystrophie.

2. Verwendung gemäß Anspruch 1 für die Herstellung eines Arzneimittels, das 25 bis 200 mg Riluzol enthält.
